# EUROPEAN PATENT APPLICATION

(11) **EP 4 360 472 A1**
(43) Date of publication of application: **01.05.2024**
(21) Application number: 22828532.6
(22) Date of filing: 24.06.2022
(51) Int. Cl.: A23L 33/11, A61K 31/352, A61P 21/00

(54) **COMPOSITION FOR SUPPRESSING MUSCLE DAMAGE**

(30) Priority: 25.06.2021 WO PCT/JP2021/024243
(71) Applicant: Otsuka Pharmaceutical Co., Ltd., Tokyo 101-8535 (JP)
(72) Inventor: OKITA, Koichi, Ebetsu-shi, Hokkaido 069-8511 (JP); IKEDA, Yasutaka, Osaka-shi, Osaka 540-0021 (JP); MIZOKAMI, Tsubasa, Osaka-shi, Osaka 540-0021 (JP); AKIYAMA, Minoru, Osaka-shi, Osaka 540-0021 (JP)
(74) Representative: Hoffmann Eitle
(86) International application number: PCT/JP2022/025267
(87) International publication number: WO 2022/270616

(57) **Abstract**

Provided is a composition for suppressing muscle damage, comprising kaempferol and/or a glycoside thereof.

## Description

### Technical Field

Disclosed are techniques related to suppression of muscle damage using kaempferol.

### Background Art

Kaempferol is a substance represented by the following structural formula and is a type of natural flavonoid found in many edible plants such as tea, broccoli, grapefruit, cabbage, kale, beans, endive, leeks, tomatoes, strawberries, grapes, Brussels sprouts, apples, quinoa, and horseradish. A study has recently reported that kaempferol ("KMP" below) improves oxygen consumption efficiency in vivo and has physiological actions such as limiting a decrease in exercise efficiency and decreasing fatigue (Patent Literature (PTL) 1).

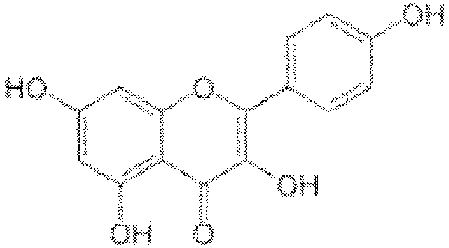

Damage to muscle (also referred to as "muscle damage") is caused by excessive exercise load or the like, leading to muscle pain and/or muscle weakness. Thus, particularly in athletes etc., muscle damage is a cause of decreased athletic performance, and there is a need to suppress and reduce muscle damage.

### Citation List

### Patent Literature

PTL 1: WO2019/044964

### Non-patent Literature

NPL 1: J. Appl. Physiol. 83(4): 1076-1082, 1997

### Summary of Invention

### Technical Problem

An object is to provide a novel means for suppressing or reducing muscle damage.

### Solution to Problem

The present inventors conducted extensive research to achieve the above object, and found that muscle damage is suppressed or reduced by using kaempferol. As a result of further research based on this finding, the invention, including the following subject matter, is provided.

### Item 1.

A composition for preventing or reducing muscle damage, comprising kaempferol and/or a glycoside thereof.

### Item 2.

The composition according to Item 1, wherein the muscle damage is accompanied by an increase in a blood myoglobin level.

### Item 3.

A composition for suppressing an increase in a blood myoglobin level, comprising kaempferol and/or a glycoside thereof.

### Item 4.

The composition according to any one of Items 1 to 3, which is a food or drink, a pharmaceutical product, or a quasi-drug.

### Item A1.

A method for preventing or reducing muscle damage, comprising allowing a subject in need thereof to ingest kaempferol and/or a glycoside thereof.

### Item A2.

The method according to Item A1, wherein the muscle damage is accompanied by an increase in a blood myoglobin level.

### Item A3.

A method for suppressing an increase in a blood myoglobin level, comprising allowing a subject in need thereof to ingest kaempferol and/or a glycoside thereof.

### Item B1.

Use of kaempferol and/or a glycoside thereof for producing a composition for preventing or reducing muscle damage.

### Item B2.

Use according to Item B1, wherein the muscle damage is accompanied by an increase in a blood myoglobin level.

### Item B3.

Use of kaempferol and/or a glycoside thereof for producing a composition for suppressing an increase in a blood myoglobin level.

### Advantageous Effects of Invention

Muscle damage can be effectively prevented, suppressed, or reduced.

### Brief Description of Drawings

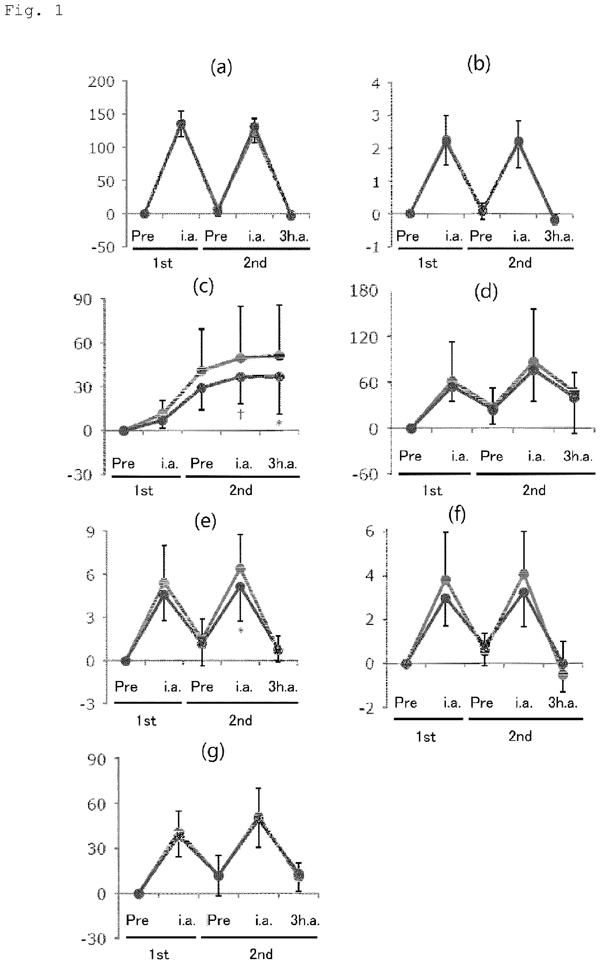

Fig. 1 shows the concentrations of various blood markers measured in the Examples. Fig. 1(a) shows the results of lactic acid, and the unit of the vertical axis is "mg/dL." Fig. 1(b) shows the results of pyruvic acid, and the unit of the vertical axis is "mg/dL." Fig. 1(c) shows the results of myoglobin, and the unit of the vertical axis is "ng/mL." Fig. 1 (d) shows the results of creatine phosphokinase (CPK), and the unit of the vertical axis is "U/L." Fig. 1(e) shows the results of aspartate aminotransferase (AST), and the unit of the vertical axis is "U/L." Fig. 1(f) shows the results of alanine aminotransferase (ALT), and the unit of the vertical axis is "U/L." Fig. 1(g) shows the results of lactate dehydrogenase (LDH), and the unit of the vertical axis is "U/L." The solid black lines are the results of ingesting kaempferol (mean ± standard deviation), and the solid gray lines are the results of ingesting a placebo (mean ± standard deviation). The symbol "†" means "P<0.1," and the symbol "*" means "P<0.05 vs placebo." "i.a." means immediately after running; "3h.a." means 3 hours after running; "1st" means 1st running;, "2nd" means 2nd running.

### Description of Embodiments

Kaempferol is a type of natural flavonoid found in many edible plants such as tea, broccoli, grapefruit, cabbage, kale, beans, endive, leeks, tomatoes, strawberries, grapes, Brussels sprouts, apples, quinoa, and horseradish. Kaempferol may be isolated, or present in an extract. In one embodiment, kaempferol is preferably derived from a plant selected from the group consisting of quinoa and horseradish.

A glycoside of kaempferol means a compound in which a sugar chain having at least one (preferably one to three, more preferably one) sugar residue is glycosidically bonded to at least one (preferably one to two, more preferably one) hydroxy group. Preferred examples of sugar residues include a glucose residue, a mannose residue, a galactose residue, a fucose residue, a rhamnose residue, an arabinose residue, a xylose residue, a fructose residue, a glucuronic acid residue, and an apiose residue. The glycoside of kaempferol can be converted to its aglycone in vivo and thus can exhibit the same action as that of kaempferol.

The composition for preventing or reducing muscle damage and the composition for suppressing an increase in the blood myoglobin level (which hereinafter may be collectively referred to as "the composition") preferably comprise kaempferol and/or a glycoside thereof as an active ingredient. In one embodiment, the composition preferably comprises kaempferol. The kaempferol and glycoside thereof may be obtained by extraction from plants according to known methods, or purchased commercially.

Muscle damage is a concept that includes muscle damage (slight damage to connective tissue surrounding myofibrils and muscle fibers) caused by excessive exercise load or the like. In one embodiment, the muscle damage is preferably muscle damage accompanied by a symptom of a blood myoglobin level. Blood myoglobin levels are known to increase with muscle damage (J. Appl. Physiol. 83(4): 1076-1082, 1997).

The amount of the kaempferol or glycoside thereof contained in the composition, the amount of the kaempferol or glycoside thereof to be taken per intake, and the amount of the kaempferol or glycoside thereof to be taken per day can be designed as appropriate within the ranges in which the desired effect is exhibited, and can be selected as appropriate according to the form of the composition, frequency of administration, health condition of the subject, and the like. The administration period of the composition is not particularly limited as long as the desired effect is exhibited, and the composition may be administered as a single dose or continuously. In one embodiment, the composition is desirably administered continuously over a certain period of time, for example, for 2 days, 3 days, 1 week, 10 days, 1 month, or 3 months or more.

In one embodiment, the composition is preferably ingested before, during, and/or after exercise (preferably exercise that may cause muscle damage). When the composition is ingested before exercise, it is preferably ingested a few hours to a few days before exercise. When the composition is ingested during exercise, it is preferably ingested between exercises (rest time). When the composition is ingested after exercise, it is preferably ingested immediately after exercise, or within a few hours or a few days after exercise. The term "a few hours" means 1 hour, 2 hours, 3 hours, 4 hours, 5 hours, 6 hours, 7 hours, 8 hours, 9 hours, 10 hours, 11 hours, or 12 hours. The term "a few days" means 1 day, 2 days, or 3 days.

The amount of the kaempferol or glycoside thereof contained in the composition may vary depending on the total weight of the composition, and is, for example, 0.1 mg to 200 mg, preferably 0.5 mg to 100 mg, more preferably 1 mg to 30 mg, and most preferably 2 mg to 15 mg, as a value in terms of kaempferol. The lower limit of the value in terms of kaempferol is, for example, 0.1 mg, 0.5 mg, 1 mg, 2 mg, or 2.5 mg, and the upper limit of the value in terms of kaempferol is, for example, 200 mg, 150 mg, 100 mg, 50 mg, 30 mg, 25 mg, or 15 mg. These upper limits and lower limits can be combined in any way.

The term "value in terms of kaempferol" means a value obtained by converting the amount of the glycoside of kaempferol into the amount of kaempferol as its aglycone. Specifically, the value in terms of kaempferol can be obtained by multiplying the number of moles of the glycoside, which is obtained by dividing the amount of the glycoside by its molecular weight, by the molecular weight of the aglycone.

The intake of the composition can be set individually based on the degree of muscle damage, age, sex, weight, diet, etc. The daily intake of the composition may be, for example, 0.1 mg to 600 mg, preferably 0.5 mg to 200 mg, and more preferably 1 mg to 100 mg, as a value in terms of kaempferol. The lower limit of the value in terms of kaempferol is, for example, 0.1 mg, 0.5 mg, 1 mg, 2 mg, or 2.5 mg. The upper limit of the value in terms of kaempferol is, for example, 600 mg, 300 mg, 200 mg, 150 mg, 100 mg, 50 mg, 30 mg, 25 mg, 15 mg, 10 mg, 5 mg, 3 mg, or 2.5 mg. These upper limits and lower limits can be combined in any way. The frequency of ingestion of the composition may be once or multiple times (e.g., 2, 3, 4, or 5 times) a day.

The composition may be a food or drink, a pharmaceutical composition, or a quasi-drug. The food or drink includes foods with function claims, foods for specified health uses, health foods, dietary supplements (supplements), foods for medical purposes, foods for the sick, and the like. The composition can be produced based on a known formulation technique and a known food manufacturing technique.

The dosage form of the composition is not particularly limited and can be designed as appropriate according to the purpose. The composition may be in the form of, for example, tablets, granules, capsules, powders, chewable tablets, confectionery (e.g., cookies, biscuits, chocolate confections, chips, cake, gum, candies, gummy candies, buns, adzuki-bean jelly, pudding, jelly, yogurt, ice cream, and sherbet), bread, noodles, cooked rice, cereal foods, drinks (e.g., liquids, soft drinks, carbonated drinks, nutritional drinks, powdered drinks, fruit drinks, dairy drinks, and jelly drinks), soups (powder, freeze-dry), miso soups (powder, freeze-dry), or other common food products.

The composition may contain any pharmaceutically acceptable carrier in addition to the kaempferol and/or glycoside thereof.

The subject that ingests the composition is not particularly limited, and is preferably a subject that may experience muscle damage. The subject that may experience muscle damage includes, for example, a subject that exercises. In one embodiment, the subject may be a human. In one embodiment, the human may be 15 years old or older, 20 years old or older, 30 years old or older, 40 years old or older, 50 years old or older, 60 years old or older, 70 years old or older, or 80 years old or older. In one embodiment, the exercise is preferably exercise such that the subject experiences muscle pain.

### Examples

The following describes the present invention in more detail with reference to Examples. However, the present invention is not limited to these Examples.

To verify the function of kaempferol, a test summarized in the table below was conducted.

**Table 1**

| | |
|---|---|
| Objective | Verification of function of KMP using 400-m run |
| Subject | 16 healthy subjects (20 to 22 years old) |
| Test substance | - Capsule food containing no KMP (placebo) |
| | - Capsule food containing 10 mg of KMP |
| Ingestion method | Single dose on the morning of the test day |
| Measurement item | Three hours after ingestion of the test substance, the subjects ran 400 m at full speed, and after a 90-minute interval, they again ran 400 m at full speed. Blood markers (lactic acid, pyruvic acid, myoglobin, CPK, AST, ALT, and LDH) were measured before, immediately after, and 3 hours after running at full speed. |

According to usual methods, blood samples were taken, and the concentration of each blood marker was measured. Fig. 1 shows the results. As shown in Fig. 1, of the blood markers measured, the blood myoglobin level after running at full speed was found to be significantly lower when KMP was ingested than that when the placebo was ingested. It is known that blood myoglobin is detected with muscle fiber damage and that its concentration increases with muscle fiber damage (J. Appl. Physiol. 83(4): 1076-1082, 1997). Thus, the results strongly suggest that muscle damage is prevented, suppressed, or reduced by the ingestion of kaempferol.

The capsule foods used in the test were prepared according to the following procedures. Quinoa grains were extracted twice with 50% aqueous ethanol at 70°C, followed by concentration under reduced pressure. To a solution obtained by purifying the resultant with resin, Aromase H2 (produced by Amano Enzyme, Inc.) was added, and the enzyme was allowed to act (conversion into aglycone) while stirring at 50°C. The resulting reaction liquid was heated to 100°C and maintained for 15 minutes to inactivate the enzyme. Thereafter, the treated liquid was freeze-dried to give an ethanol extraction powder. To the powder, starch was added in an amount equal to the amount of the powder (on a weight basis), followed by mixing. Capsules were filled with the resulting mixture in an amount of 116 mg (including 2.5 mg of kaempferol) per capsule. The subjects ingested four capsules (10 mg of kaempferol in total) at one time. The food used as the placebo was obtained by filling capsules with only starch.

## Claims

1. A composition for preventing or reducing muscle damage, comprising kaempferol and/or a glycoside thereof.

2. The composition according to claim 1, wherein the muscle damage is accompanied by an increase in a blood myoglobin level.

3. A composition for suppressing an increase in a blood myoglobin level, comprising kaempferol and/or a glycoside thereof.

4. The composition according to any one of claims 1 to 3, which is a food or drink, a pharmaceutical product, or a quasi-drug.
